# EUROPEAN PATENT SPECIFICATION

(11) **EP 1 353 717 B1**
(45) Date of publication and mention of the grant of the patent: **02.02.2005**
(21) Application number: 01981272.6
(22) Date of filing: 06.11.2001
(51) Int. Cl.: A61M 16/00

(54) **ARRANGEMENT IN VENTILATORY TREATMENT OF THE LUNGS**
ANORDNUNG ZUR VENTILATIONSBEHANDLUNG DER LUNGEN
ENSEMBLE DESTINE AU TRAITEMENT VENTILATOIRE DES POUMONS

(30) Priority: 07.11.2000 SE 0004066
(43) Date of publication of application: 22.10.2003
(73) Proprietor: Aneo AB, 195 60 Märsta (SE)
(72) Inventor: ERIKSSON, Nils, Olof, S-122 45 Enskede (SE); STRÖMBERG, Stefan, S-193 40 Sigtuna (SE)
(74) Representative: Karlsson, Leif Karl Gunnar
(86) International application number: PCT/SE2001/002436
(87) International publication number: WO 2002/038209

(56) References cited:
- EP-A2- 0 872 254
- WO-A1-98/31282
- US-A- 5 383 449
- US-A- 5 630 411

## Description

### Field of invention

The present invention relates to an arrangement for ventilatory treatment of the lungs of a living creature and has been devised primarily for the use during a state of anaesthesia, caused by intravenous infusion of a liquid anaesthesia-inducing drug as stated in the preamble of claim 1.

The invention relates more specifically to a refinement of an arrangement taught and disclosed in the European Patent Application 00 937450.5 (Arrangement in Ventilatory Treatment of the Lungs.)

### Description of the prior art

Arrangements of this kind are previously known from a number of different embodiments and are normally connected to a lung ventilator and/or unit for ventilatory treatment of the lungs.

One previously known lung ventilator, used for ventilating the lungs and for patients under a state of anaesthesia, has a first tube, running between the lung ventilator and a patient, devised for insufflation of a gas or gas mixture for the patient and a second tube connected to the lung ventilator's insufflation valve.

Lung ventilators of this kind for the stated use also have a second tube, running between the lung ventilator and a patient, with the same cross-section as the first tube. The second tube carries expired gas or a gas mixture from the patient to the lung ventilator and to an expiratory valve, connected to the tube, on the lung ventilator.

The ends of tubes, away from the lung ventilator, are joined and connected to a patient connector part for the purpose of forming a sealed, controlled path of flow for both insufflation gas and expired gas.

Connector parts of this kind are available in different versions, such as e.g. a tracheal tube and laryngeal mask.

In lung ventilators of this kind and for the aforementioned use, an insufflation valve must be open during an initial part of the insufflation phase, whereas an expiratory valve must be closed throughout the insufflation phase. The expiratory valve must be open throughout the expiratory phase, whereas the insufflation valve must be closed. An 'inspiratory pause', during which the insufflation valve and the expiratory valve are both closed, can also occur.

In this manner, the valves are both actuated in controlling the insufflation phase and the expiratory phase and are built into the lung ventilator's basic unit. Therefore, two different tubes, a first carrying insufflation gas and a second carrying expired gas, leave the lung ventilator's basic unit.

When the significant feature of the present invention is taken into consideration, it can be noted that the use of one or more gas-measuring units and one or more sensor units connected to same are previously known in anaesthesial applications.

When the measures associated with the present invention are also taken into consideration, it can be noted that in the field for utilisation of units for ventilating the lungs of a living creature which is not under a state of anaesthesia, the introduction of a control valve in the direction of ventilator gas flow and a measuring device, with a requisite sensor in the form of a device for evaluating gas flow and gas pressure, in the vicinity of the creature or patient is known from e.g. the patent publication US-A-3,961,627.

The valve's drive mechanism has e.g. a servomotor, which receives control signals from a valve control unit.

Prior art also includes the following patent publication **US-A-5 630 411.**

This patent publication discloses an apparatus for controlling the pressure of a respiratory gas delivery to a patient.

This apparatus includes a phase detection circuit for determining the inhalation and exhalation phases of a patient's respiratory cycle.

More particularly a valving apparatus according to the preamble of claim 1 is disclosed for controlling the airflow within the breathing circuit by its connection to a phase detection circuit.

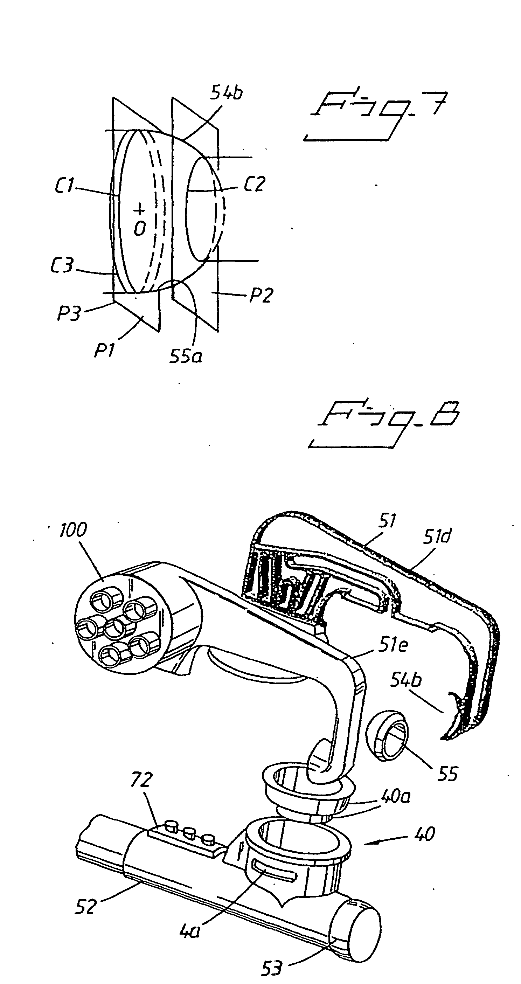

The construction of the valve is shown in Figure 5, illustrating an exploded perspective view of the major parts or components of the preferred valve arrangement or apparatus.

Thus this apparatus or control valve (14) includes a valve base (70), a shiftable or rotatable valve element (72), and a valve element cover (74).

The valve base (70) includes a housing (76) and valve motor, (78), electrically driven, and having a motor shaft (80) with locking hole (81) defined in the end thereof.

In operation pressure controller (26) energizes valve motor (78) in order to rotate element (72) clock-wise or counter clock-wise between a fully closed position (Figure 7), a fully opened position (Figure 8), and intermediate positions therebetween.

In Figures 9 to 11 is illustrated a control valve (140) of another embodiment, however also this construction is also using an electrically driven motor (156) for actuating the valve by using a pressure controller (26).

The element cover (74) and the valve motor (70) are registered by using two slots (138) and two locking bosses (106).

### Summary of the present invention

### Technical problems

When the technical deliberations that a person skilled in this particular art must make in order to provide a solution to one or more technical problems she/he encounters are taken into consideration, it will be seen that it is initially necessary to realise the measures and/or sequence or measures that must be undertaken to this end and to realise which means is/are required. On this basis, the technical problems listed below should be relevant to the development of the present invention.

When the prior art, as described above, is considered, it will be seen that a technical problem resides, in the ventilatory treatment of the lungs of a living creature under a state of anaesthesia created by intravenous infusion of a liquid anaesthesia-inducing drug, in disclosing a valve arrangement consisting of a controllable valve equipment, combined with a plurality of sensor units, which can be located very close to the creature.

It will also be seen that a technical problem resides in the provision of conditions, with simple means, enabling the arrangement and the valve equipment to offer less "dead space" in relation to known equipment for this application.

It will also be seen that a technical problem resides in realising the significance of and the advantages for a patient under a state of deep anaesthesia, induced by an intravenous infusion of a liquid drug, of being able to offer utilisation of only one insufflation tube, devised with one cross-section for being able to carry an insufflation gas or gas mixture from the lung ventilator to the patient and, additionally, a plurality of tubes with a much smaller cross-section for determining in the lung ventilator different criteria for the gas or gas mixture and for controlling a valve in said valve equipment from the lung ventilator via a lung ventilator valve arrangement

It will also be seen that a technical problem resides in realising the importance of and the advantages afforded by development of a valve arrangement made from a few simple parts, the parts being devised for easy cleaning, rinsing and/or replacement.

It will also be seen that a technical problem resides in realising the importance of and advantages afforded by having said valve equipment comprise a valve housing, enclosing sensor units, which can be made in two parts, that are attach to one another by a rotary movement. These parts can be conjoined or separated by means of this rotary movement.

It will be seen that a technical problem resides in realising the importance of and advantages afforded by arranging said rotary movement around an end-oriented valve housing's axis of rotation.

It will be seen that a technical problem resides in realising the importance of and advantages afforded by devising the area around the end-oriented axis of rotation with a passage for gas and an elastic ring-shaped gasket.

It will be seen that a technical problem resides in realising the importance of and advantages afforded by having the said elastic gasket absorb a relative, axial movement, between the said two parts forming the valve housing, in order to provide a tight seal between the respective connector means for each part of sensor unit channel tubes sections, when said parts are conjoined.

It will be seen that a technical problem resides in realising the importance of and advantages afforded by using an elastic ring-shaped gasket between said two parts. The gasket can be mounted on one of the parts and have the shape of a part or section of a sphere.

It will be seen that a technical problem resides in realising the importance of and advantages afforded by truncating said part of the sphere with two planes, located on either side of a sphere's centre, forming small circles with the same or different diameters.

It will be seen that a technical problem resides in realising the importance of and advantages afforded by having one of said two parts contain a system of channels, for interconnection to a coupling means for a plurality of tubes, preferably arranged in a single tubing bundle.

It will also be seen that a technical problem resides in realising the importance of and advantages afforded by orienting the system of channels so as to connect openings in the coupling means with the openings in one, said first, of said two parts.

It will be seen that a technical problem resides in realising the importance of and advantages afforded by equipping one of the parts with a lid, devised to seal against a peripheral area of a valve membrane.

It will be seen that a technical problem resides in realising the importance of and advantages afforded by devising one of the parts with a number of channel system first coupling means, arranged in a row, in a two-part coupling arrangement, preferably with an intermediate gasket.

It will be seen that a technical problem resides in realising the importance of and advantages afforded by devising said first coupling means on one part to enclose a second coupling means on the second part in the same coupling arrangement.

It will be seen that a technical problem resides in realising the importance of and advantages afforded by using a said ring-shaped elastic gasket with an elasticity allowing the first and second coupling means, in a coupling arrangement, to interconnect during cam-guided relative movement, between said first section or part and said second section or part.

It will be seen that a technical problem resides in realising the importance of and advantages afforded by providing one of the said two parts with a locking means, such as one or two clamps, so the first part locks firmly to the second part.

### Solution

With the intention of solving one or more of the aforesaid technical problems, the present invention takes as its starting point a valve arrangement in ventilatory treatment of the lungs of a living creature as stated in the preamble of claim 1.

The present invention proposes that said valve arrangement or equipment comprise a valve housing, which can consist of two parts. These parts can be conjoined or separated by means of an end-related rotary movement as stated in the characterizing part of claim 1.

As proposed embodiments that lie within the scope of the in entive concept, it is also proposed features as stated in the subclaims.

### Advantages

Those advantages primarily obtained by an arrangement, in accordance with the present invention, for ventilatory treatment of the lungs reside in the provision of features making possible effective co-ordination of valve equipment and an array of sensor units, so they can be placed near a living creature, preferably with the aid of a connector part. They also make possible the utilisation of only one tube for ventilatory gas between a lung ventilator unit and the living creature or patient being treated by intravenous administration of a liquid anaesthesia-inducing drug, thereby allowing expired gas from the creature to pass via the valve equipment into atmosphere in the immediate vicinity of the creature without contaminating of the lung ventilator with expired gas.

Specially shaped coupling means and a coupling arrangement that eliminate the risk of erroneous coupling are also provided. The interacting parts are also devised for easy cleaning, and a valve membrane is easily removed.

### Brief description of the drawings

The present proposed embodiment of a valve arrangement, and equipment devised for use in the ventilatory treatment of the lungs of a living creature, will now be described in greater detail with reference to the accompanying drawings, in which;
- Figure 1: is a side view of a living creature onto whose mouth and nasal area a connecting part, in the form of facemask with mounted valve equipment and a combination of sensor arrays, has been applied in accordance with the present invention's features.
- Figure 2: is a perspective view of an equipment coupling means, for connecting a bundle of tubing from a lung ventilator unit, the cross-section of the bundle of tubing between the valve equipment and the lung ventilatory unit can be regarded as evident.
- Figure 3: is a perspective cross-section side view of valve arrangement or equipment, in accordance with the present invention, with two rotating interacting parts fully conjoined.
- Figure 4: is a perspective cross-section side view of valve equipment, in accordance with fig. 3, with said two rotating, interacting parts separated.
- Figure 5: shows, on a slight enlarged scale, compared to fig. 4, the first part's end-oriented interaction with the second part's corresponding end section.
- Figure 6: shows a first and a second coupling means in a coupling arrangement in a position before being conjoined according to fig. 3.
- Figure 7: shows a sphere truncated by two planes in order to illustrate an elastic, ring-shaped gasket with a shape significant to the invention, and
- Figure 8: shows an exploded view of the valve arrangement or equipment.

### Description of embodiments at present proposed

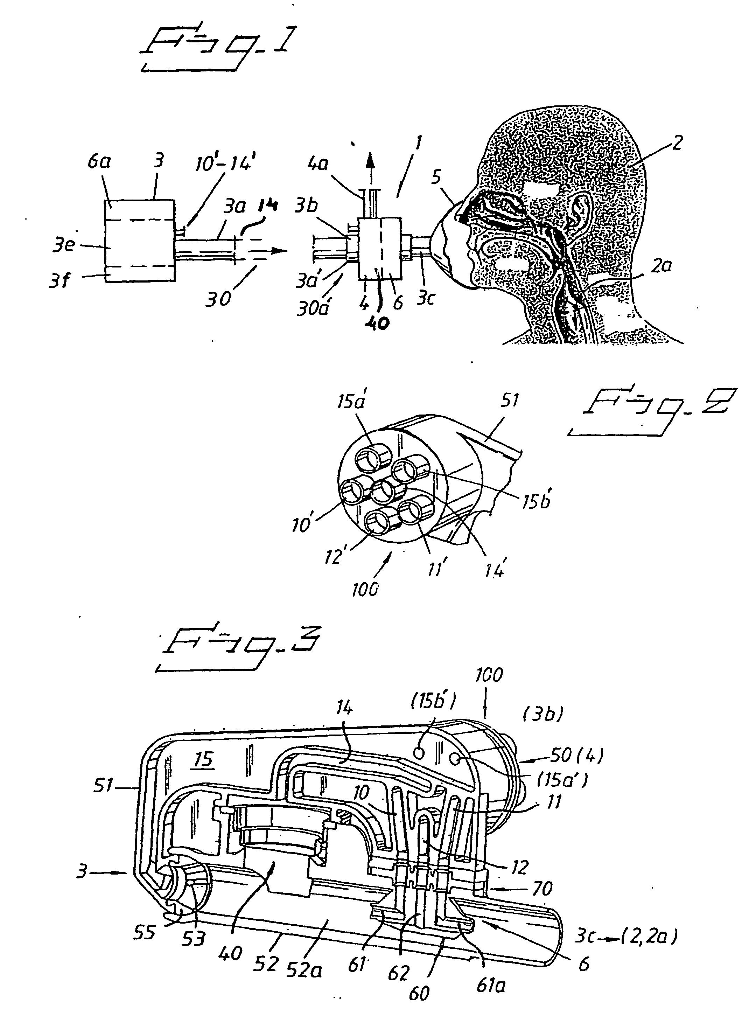

Thus, fig. 1 depicts the principles of the use of a valve arrangement 1, with a valve equipment 4 constructed in accordance with the present invention.

The arrangement 1 is devised for use in ventilatory treatment of the lungs of a living creature 2.

The ventilatory treatment of the lungs is to take place during anaesthesia induced by intravenous administration of a liquid anaesthesia-inducing drug into the patient's 2 blood.

In these circumstances, utilisation of a lung ventilation unit 3 is required as well as checks on various therapeutic and diagnostic criteria.

In accordance with the invention, only one tube or a bundle of tubing 30 leaves the lung ventilation unit 3. One tube, i.e. an insufflation tube 3a, illustrated in the embodiment example as two tubes connected in parallel to the arrangement 1, has a relatively large cross-section.

The unit 3 with requisite means for evaluating therapeutic and/or diagnostic criteria could be devised as is taught and disclosed in the European Patent Application mentioned above.

The contents of the said patent application shall accordingly be regarded as a part of the contents of the present application.

The arrangement 1 according to the present invention comprises said valve equipment 4. This equipment is devised for carrying an insufflation gas and/or an insufflation gas mixture from a lung ventilation unit 3 to the lungs and airways 2a of the living creature 2 and allowing expiration of gas and/or a gas mixture held under positive pressure in the airways.

The insufflation gas can consist of pure air, air mixed with oxygen to a greater or lesser degree or just oxygen and/or additives thereto.

This insufflation gas passes a short section of tubing 3b, the arrangement 1, valve equipment 4 and a short section of tubing 3c, in addition to the tube 3a, enters the airways 2a through a connection part 5 and descends into the lungs.

During this sequence, a valve 40 in the valve equipment 4 remains closed by pressurisation from the unit 3 via a tube 14', a coupling means and a channel 14.

When insufflation gas held in the airways 2a is expired, the expired gas is allowed to pass the said connection part 5, the section of tubing 3c and, with the aid of the valve 40 in the valve arrangement 4, through an outlet 4a into atmosphere.

Expired gas follows this route because an insufflation valve 3e in the lung ventilation unit 3 is closed, thereby preventing expired gas from passing through the section of tubing 3b and the tube 3a into the unit 3. Pressure on the valve 40 is released during this sequence or phase.

The valve equipment 4 can also be controlled in such a way that the valve equipment serves, during an insufflation phase, as a pressure relief device by having the unit 3, via a valve 3f, pressurise a tube 14' with a pre-set maximum pressure. In this manner, a pressure of e.g. 60-120 cm H₂O could activate the pressure relief device.

The valve equipment 4 is also devised to create a lower excess pressure, i.e. a PEEP pressure, of e.g. 0-30 cm H₂O, by means of reduced pressure on the tube 14' during the expiration phase.

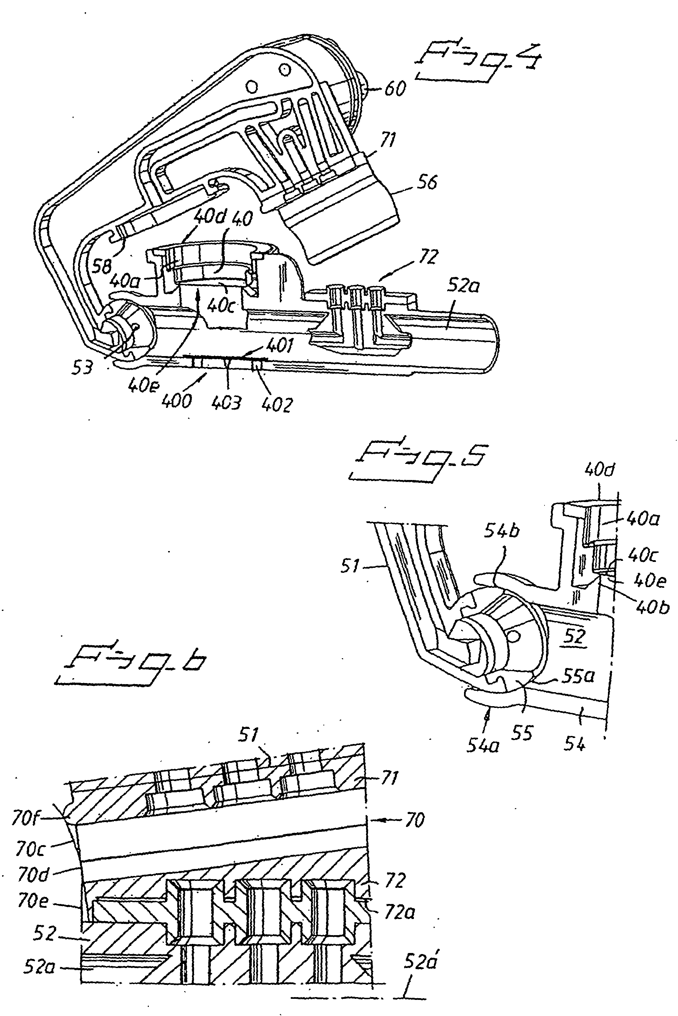

The arrangement 1 and a valve equipment 4, designated 50 in fig. 3, also has gas-measuring equipment as in a unit 3, the sensor unit or a sensor unit array 6 comprising here a plurality of sensor units in a section of tubing 52a in a second part 52. One or more gas-measuring units can advantageously be built into the lung ventilation unit 3, as shown in block 6a.

Here, the array of sensor units 6 with associated gas-measuring units in block consists of a plurality of known, co-ordinated and integrated, sensor units.

The embodiment according to fig. 3 illustrates the possibility of devising a measurement chamber 60 in the second part's section of tubing 52a, in which a plurality of sensors are combined to form said array of sensor units 6.

The array of sensor units 6 and the measurement chamber 60 incorporate and co-ordinate a plurality of sensor units in combination.

The following is a description of an example of such a combination.

One sensor 61 can accordingly be devised for measuring the velocity of insufflation gas and the duration of the insufflation phase by sensing a gas velocity-related pressure gradient.

This has been illustrated as openings 61, 61a, facing away from one another, in connection with channels 10, 11 in a first part 51 through coupling means 71, 72 (Fig. 4) in a coupling arrangement 70, the channels being connected to said tubes 10', 11' via a coupling means 100.

Here, it is assumed that the flow of gas in one direction (3b-3c) causes an increase in pressure in channel 10 and a drop in pressure in channel 11, and a momentary pressure gradient provides a measure of the momentary gas velocity.

Another sensor unit or said sensor unit 61, 61a mentioned above, can be devised for measuring the velocity of an expired gas and the duration of the expiration phase by sensing the gas velocity-related pressure gradient or a momentary pressure value and/or variation in pressure over time.

An additional sensor 62 can be devised for measuring gas pressure and/or the variation in pressure over time via a channel 12 and a tube 12'.

Gas sampling and/or pressure measurement via the channel 12 and tube 12' may be employed here.

The sensor unit 62 shall be primarily devised for measuring the presence of and/or percentage of one or more gas components in the gas mixture by diverting part of the gas mixture through the channel 12 and tube 12' for analysis.

The sensors 61, 61 a and 62 are therefore arrayed in a measurement chamber 60 for measuring velocity and pressure, sampling gas etc.

Measurement values received in the form of values, sensed via the channels 10, 11 and 12 and connected tubes 10', 11' and 12', and part of the gas mixture are sent, via the channel 12 and tube 12', to the unit 3, which contains requisite means and equipment for evaluating therapeutic and/or diagnostic criteria.

The invention is based upon the concept that the said valve equipment 50 (4) consists of two parts, i.e. a first upper part 51 and a second lower part 52. The said second part 52 contains a combination of sensor unit arrays 6 in the said measurement chamber 60, the arrays being arranged in close proximity to one another.

The tube 3a is connected by the coupling means 100 to a channel 15 by utilising two tubes 15a' and 15b' in a bundle of tubes, as shown in FIGS. 2 and 3. The said channel 15 carries a flow of air through an elastic gasket 55 and the section of tubing 52a in the second part 52.

The bundle of tubing and the tubing therein are e.g. connected to an end section 3a' of a tube 3a carrying insufflation gas from the lung ventilation unit 3, and the said array of sensor units 6 is located closer to the living creature 2 than the said valve equipment 4.

Fig. 3 shows that the said valve equipment 50(4) and the said array of sensor units 6 are integrated into a single unit, assigned the reference designation 50.

Fig. 1 shows that a bundle of tubes 30 is arranged between the equipment 4 and the unit 3.

This bundle of tubes 30 can consist of a plurality of individual tubes, attached to one another or separated, dominated by the insufflation gas tube 3a, with a large cross-section.

It is primarily proposed that all the tubes be integrated (or bundled) in a way that is evident from a study of fig. 2 and the utilised coupling means 100.

This will show that the tubes 15a' and 15b' constitute the insufflation tube (3a), that a tube 14' is a tube for controlling the expiratory valve 40, tubes 10' and 11' respectively are intended for measuring flow and a tube 12' is intended for sampling and measuring a gas, such as carbon dioxide.

Fig. 2 shows the coupling means 100 of the first part 51, and the cited tubes are obviously intended for connection to corresponding coupling means (not shown).

Therefore, five separate tube cross-sections are required for the proposed application and with the sensor units 61, 62 and 63 combined in the measurement chamber 60. These cross-sections are devised for connection to channels 10, 11, 12, 14 and 15 respectively in the equipment's 50 first part 51.

. The channels 10 and 11 are connected to the measurement chamber 60 in order to measure the velocity and duration of insufflation gas and the velocity of expired gas and duration of the expiration phase, both channels being arranged for measurement of opposite directions of flow.

The channel 14 is devised for generating varying positive pressure, ranging between two or three values, generated by the lung ventilation unit 3. These positive pressures cause the valve 40 to open at various pre-selected positive pressures on the basis of the positive pressure in a section of tubing 52a.

The valve equipment 50, in accordance with the invention, consists of said two parts, designated a first part 51 and a second part 52. These two parts 51, 52 can be conjoined, as shown in fig. 3, and separated, as shown in fig. 4, by means of a rotary movement.

The rotary movement is arranged around an axis of rotation 53, and this axis of rotation 53 is arranged at the end of a section of tubing 52a for e.g. insufflation gas.

Fig. 5 shows that the interior of the end-oriented section of tubing, assigned the reference designation 54a, has the internal shape of a part of a sphere, and this partial sphere defines an interior area 54b.

A ring-shaped gasket 55 is arranged on one of the parts illustrated as part 51, between the parts 51 and 52, and has an external shape 55a like a part of a sphere.

The partially spherical part 54a and the partially spherical part 55a are truncated by two planes located on or on either side of the centre of a sphere, forming small circles with the same or different diameters.

It also shows that one of the parts 51 has a ring-shaped lid 58, devised for sealed closure against a circular peripheral area 40d of a membrane 40a in the valve 40.

The membrane 40a is shaped like a cup with a flat lower surface 40c, a peripheral area 40e of which is devised to form a tight seal with a narrow, ring-shaped seat 40b as soon as pressure above the lower surface 40c exceeds the pressure under the lower surface 40c.

The first part 51 also has a number of first channel-closing means 71, and the second part 52 has a number of second coupling means 72 in a coupling arrangement 70, in which the said first coupling means 71 is devised to enclose the second part's 52 second coupling means 72.

The second part 52 has been equipped with a cam profile 70c, with a knee 70d located over a straight centre line 52a', perpendicular to a section of tubing 52a over a distance 70e, and the first part 51 is equipped with a guide flange 70f.

When the first part 51 and the second part 52 are fully conjoined, the guide flange 70f follows the cam profile 70c, passes the said knee 70d and joins the straight distance 70e, causing the tube parts for the channel segments to connect in parallel to the second part's 52 opposite tube parts.

The elastic ring gasket 55 absorbs this relative movement.

A flat gasket 72a, shown in fig. 6, may be used for sealing two coupling means 71, 72 in the coupling arrangement 70.

The elasticity and, in particular, the thickness of the said ring-shaped gasket 55 in the tube area 52a' of the tube 52a are such that the first and second coupling means 71, 72 are able to interconnect axially during the relative movement of the first part 51 and second part 52 back and forth.

The fig.5 also discloses that one of the said parts, i.e. the first part 51, is provided with a locking mechanism, such as one or more clamps 56, for firm connection to the second part 52.

Fig. 7 shows that a plane "P1" through a centre "O" truncates the area 54b formed by part of a sphere, thereby forming a large circle "C1", and a plane "P2" forms a small circle "C2".

The gasket 55 forming part of a sphere with its area 55a is formed by a plane "P3" in the form of a small circle C3, a small circle C2 and a small circle C3 lying on either side of the piane "P1" and a large circle C1.

However, it should be noted that the plane "P3" with the small circle C3 must be located near the large circle C1 through the plane "P1".

Fig. 8 is an exploded view of the valve equipment 4, in accordance with the invention, and the equipment's five parts (the gasket 72a excluded). Corresponding parts have been assigned the same reference designations as in the previous figures.

Fig. 8 shows that one of the parts 51 consists of two complementary conjoined parts 51d and 51e.

It should be noted that a valve 400 for spontaneous inspiration has been shown in fig. 4 with a membrane 401 which seals off a number of holes 402, and the membrane 401 is attached to the tube at a seat 403. This valve 400 can be used when required.

It will be understood that the invention is not restricted to the aforesaid exemplifying embodiment thereof and that modifications are possible within the scope as defined in the following claims.

## Claims

1. A valve arrangement (1) in the ventilatory treatment of the lungs of a living creature (2) under a state of anaesthesia, comprising valve equipment (4) devised to carry an insufflation gas and/or an insufflation gas mixture from a lung ventilation unit (3) into the lungs and airways (2a) of the living creature (2) and allow expiration of gas and/or gas mixture held in said airways, a sensor unit in gas-measuring equipment, the sensor unit (61) being devised to sense the velocity-related pressure gradient in measuring the velocity of the insufflation gas and the duration of the insufflation phase, a sensor unit (61a) devised to sense the velocity-related pressure gradient in measuring the velocity of expired gas and the duration of the expiration phase, a sensor unit (62) for measuring the pressure of the gas and its variation in pressure over time and/or a sensor unit for diverting part of the gas mixture for measuring the presence of and/or percentage of one or more gas components in the gas mixture, **characterized in that** valve equipment (50) has a valve housing consisting of at least two parts (51, 52), and these parts can be conjoined or separated by means of an end-related rotary movement arranged around an axis of rotation (53), arranged at the end of a section of a tubing (52a).

2. An arrangement according to claim 1, **characterized in that** the said rotary movement is arranged around an axis of rotation (53) at the end of a section of tubing (52a) intended for insufflation gas.

3. An arrangement according to claim 1 or 2, **characterized in that** the end of at least one section of tubing has the shape of a part of a sphere.

4. An arrangement according to claim 1, **characterized in that** a ring-shaped gasket (55) is arranged on one of the parts (51) and has the external shape of a part of a sphere.

5. An arrangement according to claim 3 or 4, **characterized in that** said part of the sphere formed by said ring-shaped gasket (55) is truncated by two planes located on either side of the centre of the sphere, forming small circles with the same or different diameters.

6. An arrangement according to claim 1, **characterized in that** one of the said two parts (51, 52) has a system of channels for connection to a means for coupling a plurality of tubes, preferably gathered in a bundle of tubing.

7. An arrangement according to claim 1 or 6, **characterized in that** one of the parts has a lid, devised for a sealed contact with a membrane in a valve located between said parts.

8. An arrangement according to claim 1, **characterized in that** one of the parts has a number of channel-closing first coupling means.

9. An arrangement according to claim 8, **characterized in that** the said first coupling means is devised to enclose said second part's second coupling means.

10. An arrangement according to claim 4, 8 or 9, **characterized in that** the elasticity of the said ring-shaped gasket is (55) such that the first and second coupling means can made to interact axially during a relative movement of said first and second part.

11. An arrangement according to claim 1, **characterized in that** one of the said parts is provided with a locking arrangement, for a firm locking to said second part when these parts are conjoined.

## Patentansprüche

1. Ventilanordnung (1) bei der künstlichen Beatmung der Lungen eines Lebewesens (2) in einem anästhesierten Zustand, welche eine Ventilvorrichtung (4) aufweist, die dazu dient, ein Insufflationsgas und/oder ein Insufflationsgasgemisch von einer Lungenbeatmungseinheit (3) in die Lungen und Luftwege (2a) des Lebewesens (2) zu tragen und das Ausatmen von Gas und/oder Gasgemisch zu ermöglichen, das in den Atemwegen enthalten ist, weiter eine Sensoreinheit in einer Gas-Messvorrichtung, wobei die Sensoreinheit (61) dazu dient, den geschwindigkeitsabhängigen Druckgradienten beim Messen der Geschwindigkeit des Insufflationsgases und der Dauer der Insufflationsphase zu messen, eine Sensoreinheit (61a), die dazu dient, den geschwindigkeitsabhängigen Druckgradienten beim Messen der Geschwindigkeit ausgeatmeten Gases und der Dauer der Ausatmungsphase zu messen, eine Sensoreinheit (62) zum Messen des Gasdrucks sowie der Druckveränderung im Laufe der Zeit und/oder eine Sensoreinheit zum Umleiten eines Teils des Gasgemischs zum Messen des Vorhandenseins und/oder des Prozentsatzes eines oder mehrerer Gas-Bestandteile in dem Gasgemisch, **dadurch gekennzeichnet, dass** die Ventilvorrichtung (50) ein ventilgehäuse aufweist, das mindestens zwei Teile (51, 52) aufweist, und diese Teile mit Hilfe einer am Ende ansetzenden Drehbewegung, die um eine Drehachse (53) gerichtet ist, welche am Ende eines Abschnitts eines Rohrs (52a) angeordnet ist, zusammengefügt bzw. getrennt werden können.

2. Anordnung nach Anspruch 1, **dadurch gekennzeichnet, dass** die Drehbewegung um eine Drehachse (53) am Ende eines Rohrabschnitts (52a) gerichtet ist, der für Insufflationsgas vorgesehen ist.

3. Anordnung nach Anspruch 1 oder 2, **dadurch gekennzeichnet, dass** das Ende mindestens eines Rohrabschnitts die Form eines Teils einer Kugel aufweist.

4. Anordnung nach Anspruch 1, **dadurch gekennzeichnet, dass** eine ringförmige Dichtung (55) auf einem der Teile (51) angeordnet ist und die Außenform eines Teils einer Kugel aufweist.

5. Anordnung nach Anspruch 3 oder 4, **dadurch gekennzeichnet, dass** der Teil der Kugel, der durch die ringförmige Dichtung (55) gebildet wird, durch zwei auf beiden Seiten des Kugelmittelpunkts positionierte Ebenen abgetrennt wird, wodurch kleine Kreise mit gleichem oder unterschiedlichem Durchmesser gebildet werden.

6. Anordnung nach Anspruch 1, **dadurch gekennzeichnet, dass** eines der beiden Teile (51, 52) ein Kanalsystem zum Anschluss an eine Einrichtung zur Verbindung einer Mehrzahl von Rohren, die bevorzugt in einem Rohrbündel angeordnet sind, aufweist.

7. Anordnung nach Anspruch 1 oder 6, **dadurch gekennzeichnet, dass** eines der Teile einen Deckel aufweist, der dazu dient, einen dichten Kontakt mit einer Membran in einem zwischen den Teilen positionierten Ventil zu schaffen.

8. Anordnung nach Anspruch 1, **dadurch gekennzeichnet, dass** eines der Teile eine Anzahl von die Kanäle verschließenden ersten Kupplungseinrichtungen aufweist.

9. Anordnung nach Anspruch 8, **dadurch gekennzeichnet, dass** die erste Kupplungseinrichtung dazu dient, die Kupplungseinrichtung des zweiten Teils zu umschließen.

10. Anordnung nach Anspruch 4, 8 oder 9, **dadurch gekennzeichnet, dass** die Elastizität der ringförmigen Dichtung (55) derart ist, dass die erste und die zweite Kupplungseinrichtung dazu veranlasst werden können, während einer relativen Bewegung zwischen dem ersten und dem zweiten Teil axial zu interagieren.

11. Anordnung nach Anspruch 1, **dadurch gekennzeichnet, dass** eines der Teile eine Einrastanordnung für ein festes Einrasten mit dem zweiten Teil, wenn die Teile zusammengefügt sind, aufweist.

## Revendications

1. Système formant valve (1) destiné au traitement ventilatoire des poumons d'un être vivant (2) sous anesthésie, comprenant un ensemble formant valve (4) conçu pour acheminer un gaz d'insufflation et/ou un mélange gazeux d'insufflation depuis une unité de ventilation pulmonaire (3) vers les poumons et les voies aériennes (2a) de l'être vivant (2) et permettre l'expiration du gaz et/ou du mélange gazeux contenu dans les voies aériennes, une unité de détection montée dans un système de mesure de gaz, l'unité de détection (61) étant conçue pour détecter le gradient de pression, lié à la vitesse, en mesurant la vitesse du gaz d'insufflation et la durée de la phase d'insufflation, une unité de détection (61a) conçue pour détecter le gradient de pression, lié à la vitesse, en mesurant la vitesse du gaz expiré et la durée de la phase d'expiration, une unité de détection (62) destinée à mesurer la pression du gaz et la variation de sa pression dans le temps et/ou une unité de détection destinée à détourner une partie du mélange gazeux afin de mesurer la présence d'un ou plusieurs composants gazeux dans le mélange gazeux et/ou leur pourcentage, **caractérisé en ce que** l'ensemble formant valve (50) comprend une cage de valve constituée d'au moins deux parties (51, 52) et **en ce que** ces parties peuvent être assemblées ou séparées par un mouvement rotatif, par rapport à une extrémité, autour d'un axe de rotation (53), prévu à l'extrémité d'une section d'un tube (52a).

2. Système selon la revendication 1, **caractérisé en ce que** le mouvement rotatif s'effectue autour d'un axe de rotation à l'extrémité d'une section d'un tube (52a) destiné au gaz d'insufflation.

3. Système selon la revendication 1 ou 2, **caractérisé en ce que** l'extrémité d'au moins une section de tube présente la forme d'une partie de sphère.

4. Système selon la revendication 1, **caractérisé en ce qu'**un joint (55) de forme annulaire est monté sur une des parties (51) et présente la forme extérieure d'une partie de sphère.

5. Système selon la revendication 3 ou 4, **caractérisé en ce que** la partie de sphère formée par le joint de forme annulaire (55) est tronquée par deux plans situés de part et d'autre du centre de la sphère, formant de petits cercles de diamètres identiques ou différents.

6. Système selon la revendication 1, **caractérisé en ce que** l'une des deux parties (51, 52) possède un système de canaux destinés à être branchés sur un moyen de couplage d'une pluralité de tubes, de préférence rassemblés en un faisceau de tubes.

7. Système selon la revendication 1 ou 6, **caractérisé en ce que** l'une des parties possède un couvercle, conçu pour permettre un contact étanche avec une membrane présente dans une valve située entre les parties.

8. Système selon la revendication 1, **caractérisé en ce que** l'une des parties possède plusieurs premiers moyens de couplage, fermant les canaux.

9. Système selon la revendication 8, **caractérisé en ce que** les premiers moyens de couplage sont conçus pour entourer les deuxièmes moyens de couplage de la deuxième partie.

10. Système selon la revendication 4, 8 ou 9, **caractérisé en ce que** l'élasticité du joint (55) de forme annulaire est telle que les premiers et deuxièmes moyens de couplage peuvent être amenés à agir axialement l'un sur l'autre lors d'un mouvement relatif de la première et de la deuxième partie.

11. Système selon la revendication 1, **caractérisé en ce que** l'une des parties est équipée d'un dispositif de verrouillage, afin d'assurer un verrouillage solide sur la deuxième partie lorsque ces parties sont assemblées.
